(19) Europäisches Patentamt / European Patent Office / Office européen des brevets

(11) **EP 2 467 497 B1**

(12) # EUROPEAN PATENT SPECIFICATION

(45) Date of publication and mention
of the grant of the patent:
**16.10.2013 Bulletin 2013/42**

(51) Int Cl.:
**C12Q 1/68** (2006.01)

(21) Application number: **10745238.5**

(22) Date of filing: **20.08.2010**

(86) International application number:
**PCT/EP2010/062144**

(87) International publication number:
**WO 2011/020906 (24.02.2011 Gazette 2011/08)**

(54) **sPLA2 IIA POLYMORPHISM ANALYSIS FOR THE DIAGNOSIS/PROGNOSIS OF A CARDIOVASCULAR DISEASE/EVENT**

ANALYSE VON SPLA2-IIA-POLYMORPHISMEN FÜR DIE DIAGNOSE/PROGNOSE VON HERZ-KREISLAUF-EREIGNISSEN/-ERKRANKUNGEN

ANALYSE DU POLYMORPHISME SPLA2 IIA POUR LE DIAGNOSTIC/PRONOSTIC D UNE MALADIE/D UN ÉVÈNEMENT CARDIOVASCULAIRE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **21.08.2009 US 235825 P**

(43) Date of publication of application:
**27.06.2012 Bulletin 2012/26**

(73) Proprietor: **INSERM (Institut National de la Santé et de la Recherche Médicale)**
**75013 Paris (FR)**

(72) Inventors:
• **MALLAT, Ziad**
**F-75015 Paris (FR)**
• **SIMON, Tabassome**
**F-75012 Paris (FR)**
• **DANCHIN, Nicolas**
**F-75015 Paris (FR)**

(74) Representative: **Cabinet Plasseraud**
**52, rue de la Victoire**
**75440 Paris Cedex 09 (FR)**

(56) References cited:
• **WOOTTON PETER T E ET AL: "Tagging-SNP haplotype analysis of the secretory PLA2IIa gene PLA2G2A shows strong association with serum levels of sPLA2IIa: results from the UDACS study." HUMAN MOLECULAR GENETICS 15 JAN 2006 LNKD- PUBMED:16368710, vol. 15, no. 2, 15 January 2006 (2006-01-15), pages 355-361, XP002607836 ISSN: 0964-6906**
• **DATABASE NCBI Probe [Online] 27 December 2005 (2005-12-27), "Sequence-specific oligonucleotide (SSO) probe for Homo sapiens variation rs11573156" XP002607837 Database accession no. Pr001824682**
• **FISCHER MARCUS ET AL: "Genetic variants within the PLA2G2A gene encoding secretory phospholipase A2 group IIA are related to the risk of myocardial infarction" Circulation, [Online] October 2006 (2006-10), XP002607838 & 79TH ANNUAL SCIENTIFIC SESSION OF THE AMERICAN-HEART-ASSOCIATION; CHICAGO, IL, USA; NOVEMBER 12 -15, 2006 American Heart Association Retrieved from the Internet: URL: http://circ.ahajournals.org/cgi/conten t/meeting_abstract/114/18_MeetingAbstracts /II_887-c? maxtoshow=&hits=10&RESULTFORMAT= &fulltext=abstract+4111&searchid=1&FIRSTIN DEX=0&volume=114&issue=18+Supplement&re sou rcetype=HWCIT>**

- **WOOTTON PETER T E ET AL: "Tagging SNP haplotype analysis of the secretory PLA2-V gene, PLA2G5, shows strong association with LDL and oxLDL levels, suggesting functional distinction from sPLA2-IIA: results from the UDACS study." HUMAN MOLECULAR GENETICS 15 JUN 2007 LNKD- PUBMED:17545304, vol. 16, no. 12, 15 June 2007 (2007-06-15), pages 1437-1444, XP002607839 ISSN: 0964-6906**
- **DIVCHEV DIMITAR ET AL: "The secretory phospholipase A2 group IIA: a missing link between inflammation, activated renin-angiotensin system, and atherogenesis?" VASCULAR HEALTH AND RISK MANAGEMENT 2008 LNKD- PUBMED:18827909, vol. 4, no. 3, 2008, pages 597-604, XP002607840 ISSN: 1176-6344**
- **KOENIG WOLFGANG ET AL: "Association between type II secretory phospholipase A2 plasma concentrations and activity and cardiovascular events in patients with coronary heart disease" EUROPEAN HEART JOURNAL (ONLINE), OXFORD UNIVERSITY PRESS, GB, US, NL LNKD- DOI:10.1093/EURHEARTJ/EHP302, vol. 30, no. 22, 7 August 2009 (2009-08-07), pages 2742-2748, XP002565770 ISSN: 1522-9645 [retrieved on 2009-08-07]**
- **SIMON TABASSOME ET AL: "Genetic Determinants of Response to Clopidogrel and Cardiovascular Events" NEW ENGLAND JOURNAL OF MEDICINE, THE, MASSACHUSETTS MEDICAL SOCIETY, WALTHAM, MA, US LNKD- DOI: 10.1056/NEJMOA0808227, vol. 360, no. 4, 22 January 2009 (2009-01-22), pages 363-375, XP002603681 ISSN: 0028-4793**
- **SIMON TABASSOME ET AL: "Impact of Secretory PLA2-IIa Gene Polymorphims on sPLA2 Activity and Cardiovascular Events Following an AMI: Results From the French Registry of Acute ST Elevation or Non-ST-elevation Myocardial Infarction (FAST-MI) Registry" Circulation, [Online] November 2009 (2009-11), XP002607841 & 82ND SCIENTIFIC SESSION OF THE AMERICAN-HEART-ASSOCIATION; ORLANDO, FL, USA; NOVEMBER 14 -18, 2009 American Heart Association Retrieved from the Internet: URL:http://circ.ahajournals.org/cgi/conten t/meeting_abstract/120/18_MeetingAbstracts /S1174-c?maxtoshow=&hits=10&RESULTFORMAT=& fulltext=abstract+5908&searchid=1&FIRSTIND EX=0&volume=120&issue=18+Supplement&res our cetype=HWCIT>**
- **EXETER H ET AL: "FUNCTIONALITY OF PLA2G2A PROMOTER VARIANTS ASSOCIATED WITH SPLA2-IIA LEVELS" ATHEROSCLEROSIS SUPPLEMENTS, ELSEVIER, vol. 11, no. 2, MS15, June 2010 (2010-06), page 112, XP027097377 ISSN: 1567-5688 [retrieved on 2010-06-01]**
- **GUARDIOLA M ET AL: "P156 PLA2G2A, PLA2G5 AND PLA2G10 VARIANTS, SPLA2 ACTIVITY AND MASS AND CHD RISK: RESULTS FROM GRACE AND EPIC-NORFOLK" ATHEROSCLEROSIS SUPPLEMENTS, ELSEVIER, vol. 11, no. 2, June 2010 (2010-06), page 49, XP027097084 ISSN: 1567-5688 [retrieved on 2010-06-01]**

**Description**

**FIELD OF THE INVENTION**

The invention is in the field of cardiovascular disease/event diagnosis and therapy. In particular, the invention relates to specific single nucleotide polymorphisms (SNPs) in the human genome and their association with cardiovascular disease.

**BACKGROUND OF THE INVENTION**

A key problem in treating vascular diseases is proper diagnosis. Often the first sign of the disease is sudden death. For example, approximately half of all individuals who die of coronary artery disease die suddenly, Furthermore, for 40-60% of the patients who are eventually diagnosed as having coronary artery disease, myocardial infarction is the first presentation of the disease. Unfortunately, approximately 40% of those initial events go unnoticed by the patient. Because of our limited ability to provide early and accurate diagnosis followed by aggressive treatment, cardiovascular diseases (CD) remain the primary cause of morbidity and mortality worldwide. Patients with CD represent a heterogeneous group of individuals, with a disease that progresses at different rates and in distinctly different patterns. Despite appropriate evidence-based treatments for patients with CD, recurrence and mortality rates remain high. Also, the full benefits of primary prevention are unrealized due to our inability to accurately identify those patients who would benefit from aggressive risk reduction.

Whereas certain disease markers have been shown to predict outcome or response to therapy at a population level, they are not sufficiently sensitive or specific to provide adequate clinical utility in an individual patient. As a result, the first clinical presentation for more than half of the patients with coronary artery disease is either myocardial infarction or death.

Physical examination and current diagnostic tools cannot accurately determine an individual's risk for suffering a complication of CD. Known risk factors such as  hypertension, hyperlipidemia, diabetes, family history, and smoking do not establish the diagnosis of atherosclerosis disease. Diagnostic modalities which rely on anatomical data (such as coronary angiography, coronary calcium score, CT or MRI angiography) lack information on the biological activity of the disease process and can be poor predictors of future cardiac events. Functional assessment of endothelial function can be non-specific and unrelated to the presence of atherosclerotic disease process, although some data has demonstrated the prognostic value of these measurements.

Individual biomarkers, such as the lipid and inflammatory markers, have been shown to predict outcome and response to therapy in patients with CD and some are utilized as important risk factors for developing atherosclerotic disease.

Nonetheless, up to this point, no single biomarker is sufficiently specific to provide adequate clinical utility for the diagnosis of CD in an individual patient.

Therefore, there is a need for identifying other factors that provide a more accurate diagnosis/ prognosis of CD.

The development of molecular biological techniques and the primary completion of the human genome project have enabled the detection of genetic variations that may be directly or indirectly related to a cardiovascular disease/ event. Thus, the invention aims to provide a novel method for the diagnosis/prognosis of CD using a genetic factor.

**SUMMARY OF THE INVENTION**

One object of the invention is a method of identifying a subject having or at risk of having or developing a cardiovascular disease and/or a cardiovascular event, comprising determining, in a sample obtained from said subject, the presence or absence of the sPLA2 type IIA haplotype comprising variant alleles in rs11573156 SNP, wherein

- haplotype wherein the variant allele (G) in SNP rs11573156 is present indicates an increased risk of having or being at risk of having or developing a cardiovascular disease and/or cardiovascular event, said haplotype being GCGAA for SNPs rs11573156, rs3753827, rs2236771, rs876018 and rs3767221 respectively.

**DETAILED DESCRIPTION OF THE INVENTION**

**<u>Definitions</u>**

"Cardiovascular disease" or "arteriovascular disease" as defined herein is a general term used to classify numerous conditions affecting the heart, heart valves, blood, and vasculature of the body and encompasses any disease affecting the heart or blood vessels, including, but not limited to, Metabolic Syndrome, Syndrome X, atherosclerosis, atherothrombosis, coronary artery disease, stable and unstable angina pectoris, stroke, diseases of the aorta and its branches (such as aortic stenosis, thrombosis or aortic aneurysm), peripheral artery disease, peripheral vascular disease, cerebrovascular disease, and including, without limitation, any acute ischemic arteriovascular event. Arteriovascular disease as used herein is meant to most commonly refer to the ischemic or pro-ischemic disease, rather than generally to-non- ischemic disease.

"Cardiovascular event" is used interchangeably herein with the term "cardiac event", "acute arteriovascular event", or "Arteriovascular event" and refers to sudden cardiac death, acute coronary syndromes such as, but not limited to, plaque rupture, myocardial infarction, unstable angina, as well as non-cardiac acute arteriovascular events such as blood clots of the leg, aneurysms, stroke and other arteriovascular ischemic events where arteriovascular blood flow and oxygenation is interrupted.

As used herein, "atherosclerosis" and "atherothrombosis" refer to systemic inflammatory disease states associated with complex inflammatory responses to multifaceted vascular pathologies involving inflammatory activation of the endothelium, inflammatory leukocytes as a source of thrombogenic stimuli, smooth muscle cells as a source of procoagulants and amplifier of the inflammatory response during thrombosis, and platelets as mediators of inflammation and thrombosis. Arteries harden and narrow due to build up of a material called "plaque" on their inner walls. As the plaque develops and increases in size, the insides of the arteries get narrower ("stenosis") and less blood can flow through them. Stenosis or plaque rupture may cause partial or complete occlusion of the affected vasculature. Tissues supplied by the vasculature are thus deprived of their source of oxygenation (ischemia) and cell death (necrosis) can occur.

"CAD" or "coronary artery disease" is an arteriovascular disease which occurs when the arteries that supply blood to the heart muscle (the coronary arteries) become atherosclerotic, calcified and/or narrowed. Eventually, blood flow to the heart muscle is reduced, and, because blood carries much-needed oxygen, the heart muscle is not able to receive the amount of oxygen it needs, and often undergoes necrosis. CAD encompasses disease states such as acute coronary syndromes (ACS), myocardial infarction (heart attack), angina (stable and unstable), and atherosclerosis and atherothrombosis that occurs in the blood vessels that supply the heart with oxygen-rich blood. An estimated 13 million Americans are currently diagnosed with CAD, with approximately 7 million being the survivors of past acute events. Over 1 million new acute CAD events occur each year, many resulting in death. The lifetime risk of CAD after age 40 is 49 percent for men and 32 percent for women. Subjects who are deemed clinically to be at low risk or no risk for developing arteriovascular disease such as CAD often exhibit none or few of the traditional risk factors for the arteriovascular disease, but nevertheless may still be at risk for an acute arteriovascular event. Approximately 20% of all acute CAD events occur in subjects with none of the traditional risk factors, and the majority of all acute CAD occur in subjects who have not been previously diagnosed with CAD. Often these subjects do not exhibit the symptoms of an acute CAD event, i.e. shortness of breath and/or chest pain, until the actual occurrence of such an acute event. A substantial detection gap remains for those who are at risk for an acute CAD event yet are asymptomatic, without traditional risk factors, or are currently deemed clinically to be at low risk and have not yet been diagnosed with CAD.

"CVD" or "cerebrovascular disease" is an arteriovascular disease in the blood vessels that feed oxygen-rich blood to the face and brain, such as atherosclerosis and atherothrombosis. This term is often used to describe "hardening" of the carotid arteries, which supply the brain with blood. It is a common comorbid disease with CAD and/or PAD. It is also referred to as an ischemic disease, or a disease that causes a lack of blood flow. CVD encompasses disease states such as cerebrovascular ischemia, acute cerebral infarction, stroke, ischemic stroke, hemorrhagic stroke, aneurysm, mild cognitive impairment (MCI) and transient ischemic attacks (TIA). Ischemic CVD is believed to closely relate to CAD and PAD; non-ischemic CVD may have multiple pathophysiologies. An estimated 5 million Americans are the survivors of past diagnosed acute CVD events, with an estimated 700 thousand acute CVD events occurring each year. As disclosed herein, subjects deemed to be at low risk or no risk of CVD based on clinical assessments of traditional arteriovascular disease risk factors, or without symptoms such as TIAs, MCI or severe headache, may still be at risk for an acute CVD event.

"PAD" or "peripheral artery disease" encompasses disease states such as atherosclerosis and atherothrombosis that occur outside the heart and brain. It is a common comorbid disease with CAD. Subjects who are deemed to be at low risk or no risk of PAD based upon an assessment of traditional risk factors of PAD (or arteriovascular disease), or who are asymptomatic for PAD or an arteriovascular disease may nevertheless be at risk for an arteriovascular event, even in the absence of claudication. Claudication can be defined as pain or discomfort in the muscles of the legs occurring due to a decreased amount of blood flowing to a muscle from narrowing of the peripheral arteries, producing ischemia and often arterial occlusion, causing skeletal muscle and limb necrosis. The pain or discomfort often occurs when walking and dissipates under resting conditions (intermittent claudication). Pain, tightness, cramping, tiredness or weakness is often experienced as a result of claudication. PAD not only causes the hemodynamic alterations common in CAD, but also results in metabolic changes in skeletal muscle. When PAD has progressed to severe chronic and acute peripheral arterial occlusion, surgery and limb amputation often become the sole therapeutic options. PAD is widely considered to be an underdiagnosed disease, with the majority of confirmed diagnoses occurring only after symptoms are manifested, or only with other arteriovascular disease, and irreversible arteriovascular damage due to such ischemic events has already occurred.

"Cardiovascular Risk Factor" encompasses one or more biomarker whose level is changed in subjects having a cardiovascular disease or is predisposed to developing a cardiovascular disease, or at risk of a cardiovascular event.

"Risk" in the context of the present invention, relates to the probability that an event will occur over a specific time period, as in the conversion to arteriovascular events, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1-p) is the probability of no event) to no-conversion. Alternative continuous measures which may be assessed in the context of the present invention include time to arteriovascular disease conversion and therapeutic arteriovascular disease conversion risk reduction ratios. "Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition to an arteriovascular condition or to one at risk of developing an arteriovascular event, or from at risk of an arteriovascular event to a more stable arteriovascular condition. Risk evaluation can also comprise prediction of future clinical parameters, traditional laboratory risk factor values, or other indices of arteriovascular disease, such as coronary calcium scores, other imaging or treadmill scores, passive or provocative testing results, arteriovasculature percentage stenosis or occlusion and other measurements of plaque burden and activity, either in absolute or relative terms in reference to a previously measured population. The methods of the present invention may be used to make continuous or categorical measurements of the risk of conversion to arteriovascular disease and events, thus diagnosing and defining the risk spectrum of a category of subjects defined as being at risk for an arteriovascular event. In the categorical scenario, the invention can be used to discriminate between normal and other subject cohorts at higher risk for arteriovascular events. In other embodiments, the present invention may be used so as to discriminate those at risk for developing an arteriovascular event from those having arteriovascular disease, or those having arteriovascular disease from normal.

A "sample" in the context of the present invention is a biological sample isolated from a subject and can include, by way of example and not limitation, bodily fluids and/or tissue extracts such as homogenates or solubilized tissue obtained from a subject. Tissue extracts are obtained routinely from tissue biopsy and autopsy material. Bodily fluids useful in the present invention include blood, urine, saliva or any other bodily secretion or derivative thereof. As used herein "blood" includes whole blood, plasma, serum, circulating epithelial cells, constituents, or any derivative of blood.

"Clinical parameters or indicia" encompasses all non-sample or non-analyte biomarkers of subject health status or other characteristics, such as, without limitation, age (Age), ethnicity (RACE), gender (Sex), diastolic blood pressure (DBP) and systolic blood pressure (SBP), family history (FamHX), height (HT), weight (WT), waist (Waist) and hip (Hip) circumference, body-mass index (BMI), as well as others such as Type I or Type II Diabetes Mellitus or Gestational Diabetes Mellitus (DM or GDM, collectively referred to here as Diabetes), and resting heart rate.

A "subject" in the context of the present invention is preferably a mammal. The mammal can be a human, non-human primate, mouse, rat, dog, cat, horse, or cow, but are not limited to these examples. Mammals other than humans can be advantageously used as subjects that represent animal models of arteriovascular disease or arteriovascular events. A subject can be male or female. A subject can be one who has been previously diagnosed or identified as having arteriovascular disease or an arteriovascular event, and optionally has already undergone, or is undergoing, a therapeutic intervention for the arteriovascular disease or arteriovascular event. Alternatively, a subject can also be one who has not been previously diagnosed as having arteriovascular disease. For example, a subject can be one who exhibits one or more risk factors for arteriovascular disease, or a subject who does not exhibit arteriovascular risk factors, or a subject who is asymptomatic for arteriovascular disease or arteriovascular events. A subject can also be one who is suffering from or at risk of developing arteriovascular disease or an arteriovascular event.

The term "Allele" has the meaning which is commonly known in the art, that is, an alternative form of a gene (one member of a pair) that is located at a specific position on a specific chromosome which, when translated result in functional or dysfunctional (including non-existent) gene products.

The term "polymorphism" or "allelic variant" means an alteration in the normal sequence of a gene. Allelic variants can be found in the exons, introns, or the coding region of the gene, or in the sequences that control expression of the gene. Complete gene sequencing often identifies numerous allelic variants (sometimes hundreds) for a given gene. The significance of allelic variants is often unclear until further study of the genotype and corresponding phenotype occurs in a sufficiently large population.

The term "Single nucleotide polymorphism" or "SNP" means a single nucleotide variation in a genetic sequence that occurs at appreciable frequency in the population. There are millions of SNPs in the human genome. Most commonly, these variations are found in the DNA between genes. When SNPs occur within a gene or in a regulatory region near a gene, they may play a more direct role in disease by affecting the gene's function.

The term "linkage disequilibrium" (LD) refers to a population association among alleles at two or more loci. It is a measure of co-segregation of alleles in a population. Linkage disequilibrium or allelic association is the preferential association of a particular allele or genetic marker with a specific allele, or genetic marker at a nearby chromosomal location more frequently than expected by chance for any particular allele frequency in the population.

The term "haplotype" means a 5' to 3' sequence of nucleotides found at a set of one or more polymorphic sites in a locus on a single chromosome from a single individual. The term "polymorphic site" refers to a region in a nucleic acid at which two or more alternative nucleotide sequences are observed, often in a significant number of nucleic acid samples from a population of individuals.

The term "locus" as it is used herein refers to any specific region of DNA, a gene, a group of genes or any group of nucleotides defining a DNA region of interest.

The term "sPLA2 type IIA" refers to the member IIA of the secreted phospholipases A2 (sPLA2) family of enzymes that hydrolyze phospholipids at the sn-2 position to release free fatty acids and lysophospholipid. In humans, nine catalytically-active sPLA2s (IB, IIA, IID, IIE, IIF, III, V, X and XIIA) and two catalytically-inactive sPLA2-like proteins (XIIB and otoconin-95) have been identified. sPLA2 type IIA has been the most characterized enzyme.

As used herein, the expression "sPLA2 type IIA activity" refers to the ability of the sPLA2 type IIA of metabolizing oxidized phospholipids (OxPL) by cleaving the oxidized fatty acid side chain at the sn2 position of OxPL to generate lysophosphatidylcholine and an oxidized free fatty acid.

The measurement of the quantity (or mass) of sPLA2 type IIA can be performed following routine techniques, well known by the person skilled in the art. For example, said measurement may be performed by an immunoassay allowing the measurement of sPLA2 type IIA concentrations in cell culture supernatant, serum and plasma. Said immunoassay is based on a double-antibody "sandwich" technique. Commercial kits for carrying out this immunoassay are available on the market. For instance, the quantity of sPLA2 type IIA may be performed with sPLA2 EIA Kit commercialised by Cayman. This immunoassay is based on a double-antibody "sandwich" technique. An antibody specific for sPLA2 has been pre-coated onto a microplate. Standards and samples are pipetted into wells and any sPLA2 type IIA present is bound by the immobilized antibody. An enzyme-linked antibody specific for sPLA2 is also added to the wells. The "sandwiches" are immobilized on the plate so that the excess reagents may be washed away. Following this step of washing, a substrate solution is added to the wells and color develops in proportion to the amount of sPLA2 bound in the initial step. The color development is stopped and the intensity of the color is measured.

The measurement of sPLA2 type IIA activity can be performed by a fluorimetric assay according to Radvanyi et al. (1989 Anal Biochem 177: 103- 9) as modified by Pernas et al. (1991 Biochem Biophys Res Commun 178: 1298-1305) . In particular, the following assay is used. The 1- hexadecanoyl- 2- (1- pyrenedecanoyl)- sn- glycero- 3- phosphomethanol sodium salt (Interchim, Montlucon, France) is used as a substrate for sPLA2. The hydrolysis of this substrate by sPLA2 yields 1- pyrenedecanoic acid, which emits fluorescence at 397 nm. A volume (E) of 0.03 ml of the aliquoted plasmas is mixed with 5 nmol of substrate in presence of a 10 mM Tris- HCL pH 8.7, 0.1 % albumin, 10 mM CaCl2 in a total volume of 2.5 ml, and fluorescence (F) is measured at 397 nm after one minute. 100% hydrolysis of the substrate is obtained with 0.1 U of bee venom PLA2 (Sigma Chemical Co., France) during one minute, the value of the fluorescence at the end of the one minute reaction (Fmax) thus corresponds to an activity of 2 nmoles/min/ml (Vmax) . The activity (A) of the sample (expressed in nmol/ml/min) is given by the following formula: A = (Vmax*F) / (E*Fmax) . The samples are diluted when substrate hydrolysis is above 50%. The hydrolysis of substrate in the absence of plasma is used as negative control and deduced from sPLA2 activity. All samples are tested in duplicate. The minimum detectable activity and detection limit is 0.10 nmole/min/ml and the intra and inter assay coefficient of variation is lower than 10%.

The measurement of sPLA2 activity can be performed by an improved fluorimetric assay, using an automated fluorimetric measurement, with a small sample volume, a modified substrate/ enzyme ratio (10 nmoles/U instead of 50 nmoles/U) and a thermostat ruled at 30°C, providing a higher precision and sensitivity (2, 7 % < within batch coefficient of variation (CV) < 3, 2%) and between batch CV= 5, 7%) than the previous method (within batch CV<10% and between batch CV<10%) and a substantially shorter time to complete the assay. In particular, the following assay is used for automated measurement. The 1- hexadecanoyl- 2- (1- pyrenedecanoyl)- sn- glycero- 3- phosphomethanol sodium salt (Interchim, Montlucon, France) is used as a substrate for sPLA2. The hydrolysis of this substrate by sPLA2 yields 1- pyrenedecanoic acid, which emits fluorescence at 405 nm. Briefly, 1 nmol of fluorescent substrate in 0.2 ml of buffer substrate (10 mM Tris- HCL pH 8.7, 0.1 % albumin, 10 mM CaCl2) was automatically distributed in Black Maxisorp microtitration plate (96 wells) . Because the self- quenching properties of the substrate, a low fluorescence is firstly recorded (Fmin) in a Fluostar Optima fluorimeter equipped with a stirring device and thermostat ruled at 30°C. The addition of 30 µl (100 U/mL) of bee venom PLA2 (Sigma Chemical Co., France) leads to a rapid hydrolysis of all substrate (100% of hydrolysis) and an increase in fluorescence to a maximal value (Fmax), corresponding to an activity of 5 nmol/ml. To determine the sPLA2 activity in unknown blood samples, 30 µl of sera (E) were automatically distributed and added to the substrate mixture and the fluorescence was recorded at one minute (F) . A two- point procedure was used to measure the corrected fluorescence intensity of each sample and to evaluate the enzymatic activity (expressed in nmol/min/ml) . All samples were tested in duplicate. The activity (A) of the sample (expressed in nmol/ml/min) is given by the following formula: A = (Vmax*F) / (E*Fmax) . The hydrolysis of the substrate in the absence of serum is used as negative control and deduced from PLA2 activity. All samples are tested in duplicate. Unless otherwise mentioned, all the numerical values given herein for serum sPLA2 activity are measured according to the above defined assay for automated measurement. The phospholipase that can be used to perform the assay is a secretory phospholipase or a phospholipase with a known activity and preferably a bee venom phospholipase.

The sPLA2 activity may be determined by a process based on a fluorimetric assay comprising contacting a biological sample containing said sPLA2 and taken from said patient, with a substrate at a concentration from 1 nM to 15 nM, the serum sample volume being from 5µl to 50 µl and the substrate volume being from 100µl to 300µl, at a temperature range from about 15°C to about 40°C and preferably 30°C. The phospholipase used could be a phospholipase from bee venom or snake venom like Naja venom, preferably bee venom. It could be a recombinant phospholipase from any species. This assay is described in example 2 of WO2008/015546. The advantage of this method is the small sample volume of substrate used and the thermostating, providing a higher precision and sensitivity.

Alternatively, a variant of the automated fluorimetric measurement as defined above can be used, which enables to alleviate imprecision which might result from a non- specific increase in fluorescence intensity due to other factors in the sample, thus interfering with the measure of sPLA2 activity. This method only differs from the above- defined automated fluorimetric measurement method in that the following formula is used for determining sPLA2 activity:

$$A = F*s/[(Fmax-Fmin)*V]$$

wherein:

- A represents sPLA2 activity expressed in nmol/min/ml;
- s represents the quantity of substrate expressed in nmoll (usually 1 nmol in a volume of 200 µl of working solution);
- V represents the sample volume expressed in ml (usually from 0.30 to 0.50 ml);
- (Fmax - Fmin) represents the difference between the maximal fluorescence signal at the end of the reaction in the presence of PLA2 from bee venom and the negative control;
- F represents the initial slope, within linear range, of the curve representing fluorescence emission as a function of time, expressed in $min^{-1}$.

This variant of the automated fluorimetric measurement is described in example 4 of WO2008/015546.

## The Invention

Secretory phospholipase $A_2$ (sPLA$_2$) enzymes, including type IIA, V and X, are believed to play important roles in several atherogenesis-related pathways, ranging from lipoprotein modification and foam-cell formation to the production of proinflammatory bioactive lipids that perpetuate vascular inflammation[1-4].

Elevated circulating levels of sPLA$_2$ type IIA mass and sPLA$_2$ activity, which result from several types of circulating sPLA$_2$ enzymes including type IIA, are associated with an increased incidence of future coronary artery disease in apparently healthy individuals[5,6] and with adverse outcomes in smaller populations of patients with stable[7] or unstable coronary artery disease[8].

The sPLA2 type IIA gene comprises several SNPs. While studying the association between five SNPs (rs11573156, rs3753827, rs2236771, rs876018 and rs3767221) of PLA2 type IIA gene polymorphism, sPLA2 activity and the occurrence of cardiovascular events, the inventors surprisingly found that specific genetic variations in the sPLA2 type IIA gene are associated with sPLA2 activity and the diagnosis/prognosis of a cardiovascular disease/event.

This finding suggests a causal role of sPLA2 activity in cardiovascular disease/event. In addition, this finding also strongly supports the clinical testing of sPLA2 inhibitors to limit the progression and complications of ischaemic cardiovascular disease.

The five SNPs studied in the sPLA2 type IIA gene are described here after:

| rs ID | hCV ID | Chr | position build v36 | Gene | Transcription Orientation | Ac Am Change |
|---|---|---|---|---|---|---|
| rs11573156 | hCV31442908 | 1 | 20 178 733 | PLA2G2A | - | 5'UTR |
| rs3753827 | hCV73550 | 1 | 20 178 474 | PLA2G2A | - | intronic |
| rs2236771 | hCV16195973 | 1 | 20 177 549 | PLA2G2A | - | syn (32T32) |
| rs876018 | hCV12011400 | 1 | 20 174 514 | PLA2G2A | - | Downstream |
| rs3767221 | - | 1 | 20 174 368 | PLA2G2A | - · | Downstream |

| rs ID | dbSNP sequence (5'->3') | Allele 1 (dbSNP) | Allele 2 (dbSNP) |
|---|---|---|---|
| rs11573156 | CCTACCCCCAACCTCCCAGAGGGAG[C/G]AGC TATTTAAGGGGAGCAGGAGTGC (SEQ ID NO : 1) | G | C |
| rs3753827 | CACATACACACACTCTCGTA[A/C]TTACCTGAT AGTGCCAACAT (SEQ ID NO : 2) | A | C |
| rs2236771 | CACAGAATGATCAAGTTGAC[C/G]ACAGGAAA GGAAGCCGCACT (SEQ ID NO : 3) | G | C |
| rs876018 | GCATACACACACACATATAC[A/T]TGATTTGC TAATTGCTTTAT (SEQ ID NO : 4) | A | T |
| rs3767221 | TTCTGTGAGCTCAAGCAATC[A/C]TTGCACTTC AGCCTCAGCCT (SEQ ID NO : 5) | A | C |

A first object of the invention is a method of identifying a subject having or at risk of having or developing a cardiovascular disease and/or a cardiovascular event, comprising determining, in a sample obtained from said subject, the presence or absence of the sPLA2 type IIA haplotype comprising variant alleles in rs11573156 SNP, wherein - haplotype wherein the variant allele (G) in SNP rs11573156 is present indicates an increased risk of having or being at risk of having or developing a cardiovascular disease and/or cardiovascular event, said haplotype being GCGAA for SNPs rs11573156, rs3753827, rs2236771, rs876018 and rs3767221 respectively.

The presence of the minor allele (G) of SNP rs 11573156 indicates an increased risk of having or being at risk of having or developing a cardiovascular disease and/or cardiovascular event. As shown in the examples, the presence of the minor allele SNP rs11573156 is significantly associated with an increase in sPLA2 activity compared to subjects that do not exhibit said SNP.

The presence of minor allele (C) in SNP rs2236771 indicates a decreased risk of having or being at risk of having or developing a cardiovascular disease and/or cardiovascular event. As shown in the examples, the presence of minor allele SNP rs2236771 is significantly associated to a decrease in sPLA2 activity compared to subjects that do not exhibit said SNP.

Of the 30 potential haplotypes defined by the five SNPs present in the sPLA2 type IIA gene, the inventors also found that

i) haplotype in which the variant allele (minor allele G) in SNP rs11573156 is present is significantly associated with an increase in sPLA2 activity compared to the most frequent haplotype, and

ii) haplotype in which the variant allele (minor allele C) in SNP rs2236771 is present is significantly associated with a decrease in sPLA2 activity compared to the most frequent haplotype.

Also disclosed is a method of identifying a subject having or at risk of having or developing a cardiovascular disease and/or a cardiovascular event, comprising determining, in a sample obtained from said subject, the presence or absence of the sPLA2 type IIA haplotype comprising variant alleles in rs11573156 or rs2236771 SNPs, wherein

- haplotype wherein the variant allele (G) in SNP rs11573156 is present indicates an increased risk of having or being at risk of having or developing a cardiovascular disease and/or cardiovascular event, and
- haplotype wherein the variant allele (C) in SNP rs2236771 is present indicates a decreased risk of having or being at risk of having or developing a cardiovascular disease and/or cardiovascular event.

The presence of haplotype in which the variant allele in SNP rs11573156 is present indicates an increased risk of having or being at risk of having or developing a cardiovascular disease and/or cardiovascular event. As shown in the examples, the presence of the GCGAA haplotype, wherein G represents the variant allele of rs11573156, is significantly associated with an increase in sPLA2 activity and with an increased risk of development of cardiovascular disease/ event, independently of classic cardiovascular risk factors, compared to subjects that do not exhibit said haplotype.

The presence of the haplotype in which the variant allele in SNP rs2236771 is present indicates a decreased risk of having or being at risk of having or developing a cardiovascular disease and/or cardiovascular event. As shown in the examples, the presence of the CCCAC haplotype, wherein C represents the variant allele of SNP rs2236771, is significantly associated to a decrease in sPLA2 activity and is associated with a decreased risk of development of cardiovascular disease/event, compared to subjects that do not exhibit said SNP.

In one embodiment of the invention, the subject having or being at risk of having or developing a cardiovascular disease/event may be a substantially healthy subject, which means that the subject has not been previously diagnosed or identified as having or suffering from a cardiovascular disease, or that has not developed a cardiovascular event.

In another embodiment, said subject may also be one that is asymptomatic for the cardiovascular disease. As used herein, an "asymptomatic" subject refers to a subject that do not exhibit the traditional symptoms of a cardiovascular disease or event, including, but not limited to, chest pain and shortness of breath for CAD, claudication for PAD, and TIAS, MCI and severe headache for CVD.

In another embodiment of the invention, said subject may be one that is at risk of having or developing a cardiovascular disease or cardiovascular event, as defined by clinical indicia such as for example: age, gender, LDL concentration, HDL concentration, triglyceride concentration, blood pressure, body mass index, CRP concentration, coronary calcium score, waist circumference, tobacco smoking status, previous history of cardiovascular disease, family history of cardiovascular disease, heart rate, fasting insulin concentration, fasting glucose concentration, diabetes status, and use of high blood pressure medication.

In another embodiment of the invention, said subject may be one that has been previously diagnosed or identified for a cardiovascular disease or cardiovascular event, such as for example chronic ischemic disorders without myocardial necrosis (for example stable or effort angina pectoris), acute ischemic disorders without myocardial necrosis (for example unstable angina pectoris), ischemic disorders with myocardial necrosis (for example ST segment evaluation myocardial infarction or non-ST segment elevation myocardial infarction).

Tissue ischemia is often defined in relative terms and occurs when the needs in oxygen exceed the delivery of oxygen to tissues. There is an imbalance between tissue (myocardial for example) oxygen demands and supply. This condition of oxygen deprivation may be accompanied by inadequate removal of metabolites consequent to reduced perfusion. Myocardial ischemia can be diagnosed clinically (chest pain for example), biologically (increase in myeloperoxidase activity for example), metabolically, using scintigraphy, by analyzing regional wall motion disorders or by use of an electrocardiogram (typical modifications of the ST segment, upper or lower ST segment deviation, typical changes in T wave such as T wave inversion or steep symmetric or high amplitude positive T waves). Silent ischemia is typically diagnosed using scintigraphy or a 24h electrocardiogram recording.

Stable and effort angina is typically manifested by a chest pain during exercise and slowly recovers at rest. It usually reflects tissue ischemia during exercise.

Unstable angina is a recent increase in the frequency and/or severity of stable angina, a first episode of angina, or an angina at rest.

Myocardial necrosis is typically diagnosed by an increase in myocardial enzymes (for example troponin I, troponin T, CPK) in the circulating blood.

In another embodiment of the invention, said subject may be one who shows an improvement in cardiovascular risk factors as a result of treatments and/or therapies for cardiovascular diseases. Such improvements include a reduction in body mass index, a reduction in total cholesterol, a reduction in LDL levels, an increase in HDLC levels, a reduction in systolic and/or diastolic blood pressure, or other aforementioned risk factor or combinations thereof.

In one embodiment of the invention, no onset of ischemic symptom has been diagnosed in the subject. Myocardial ischemia can be diagnosed clinically (chest pain for example), biologically (increase in myeloperoxidase activity for example), metabolically using scintigraphy, by analysing regional wall motion disorders or by use of an electrocardiogram (typical modifications of the ST segment, upper or lower ST segment deviation, typical changes in T wave such as T wave insertion or steep symmetric or high amplitude positive T waves).

In another embodiment, an onset of ischemic symptoms has been diagnosed in the subject.

In one embodiment of the invention, the subject is a mammal, preferably a human.

In another embodiment of the invention, the sample obtained from the subject comprises bodily fluids (such as blood, saliva or any other bodily secretion or derivative thereof), and/or tissue extracts such as homogenates or solubilized tissue obtained from the subject. In a preferred embodiment, the sample to be tested is blood.

According to the invention, the sample comprises sPLA2 type IIA nucleic acid, wherein the sPLA2 type IIA nucleic acid may be genomic DNA, heterogenous nuclear RNA (hnRNA, also referred as incompletely processed single strand of ribonucleic acid) and/or cDNA.

According to the invention, the determination of the presence or absence of said SNPs may be determined by nucleic acid sequencing, PCR analysis or any genotyping method known in the art. Examples of such methods include, but are not limited to, chemical assays such as allele specific hybridation, primer extension, allele specific oligonucleotide ligation, sequencing, enzymatic cleavage, flap endonuclease discrimination; and detection methods such as fluorescence, chemiluminescence, and mass spectrometry.

Also disclosed is a kit for identifying whether a subject has or is at risk of having or developing a cardiovascular disease and/or a cardiovascular event, comprising:

- at least one primer and/or at least one probe for amplification of a sequence comprising a SNP selected from the group consisting of rs11573156 and rs2236771, or at least one primer and/or at least one probe for amplification of a sequence which allows the determination of the haplotype defined by the SNPs of the sPLA2 type IIA gene and
- instructions for use.

The primer or probe may be labelled with a suitable marker. The primer or probe may be coated on an array.

Also disclosed is a method for predicting the medical response of a subject having or developing a cardiovascular disease and/or a cardiovascular event, to a drug decreasing the quantity and/or inhibiting the activity of sPLA2 type IIA. Indeed, the inventors have shown that the presence of the minor allele SNP rs11573156 is associated with an increased risk of having or developing a cardiovascular disease and/or a cardiovascular event. Accordingly, disclosed herein is a mean by which a physicist may predict the reaction of a patient if subjected to a treatment which decreases the quantity and/or inhibits the activity of sPLA2 type IIA. Disclosed herein is a mean by which a physicist may predicts the reaction of a patient subjected to a treatment which inhibits the activity of sPLA2 type IIA.

Also disclosed is a method for predicting the responsiveness of a subject at risk of having or developing a cardiovascular disease and/or a cardiovascular event, to a drug decreasing the quantity and/or inhibiting the activity of sPLA2 type IIA, said method comprising a step of determining if the minor allele (G) of SNP rs 11573156 is present or if the haplotype wherein the variant allele (G) in SNP rs 11573156 is present, wherein said presence of the minor allele (G) of SNP rs 11573156 or said presence of the haplotype wherein the variant allele (G) in SNP rs 11573156 is present are indicative of responsiveness of the patient to said drug.

## DESCRIPTION OF THE FIGURES

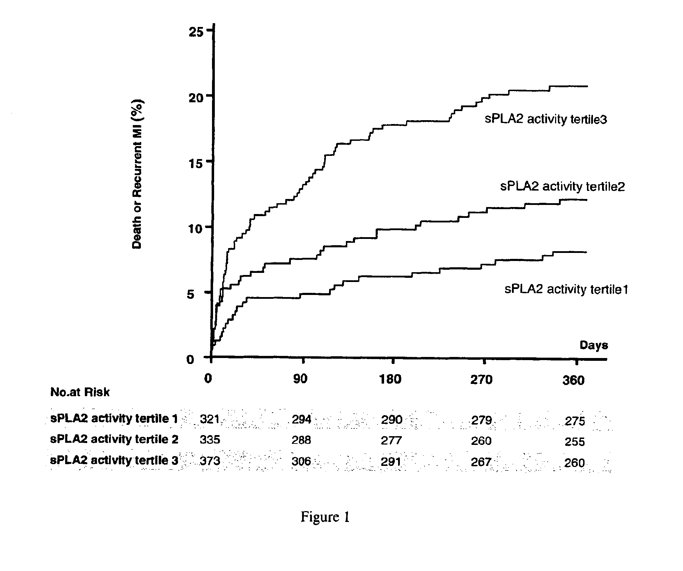

**Figure 1:** Probability of outcome events as a function of baseline sPLA2 activity.

**Figure 2:**

> (A) Map of the sPLA2 type IIA gene showing the exons (filled boxes) and introns (hatched boxes) and the position of the five SNPs, numbered from the start of exon 1.
> (B) HAPLOVIEW LD (D') display of the five SNPs. The darker the box the stronger the LD. The D' LD for any two SNPs is presented in the box representing their intersection. No number denotes complete LD.

**EXAMPLES**

**Methods**

*Study population*

The population and methods of the French registry of Acute ST-elevation and non-ST-elevation Myocardial Infarction (FAST-MI) have been described in detail elsewhere.[11] Briefly, the objective of the FAST-MI registry was to gather complete and representative data on the management and outcome of patients admitted to intensive care units for definite acute MI, irrespective of the type of institution to which they were admitted (i.e., university hospitals, public hospitals, or private clinics). All consecutive adult patients ($\geq$18 years) were included in the registry if they had elevated serum markers of myocardial necrosis higher than twice the upper limit of normal for creatine kinase, creatine kinase-MB or elevated troponins, and either symptoms compatible with acute MI and/or electrocardiographic changes on at least two contiguous leads with pathologic Q waves ($\geq$0·04 sec) and/or persisting ST elevation or depression >0.1 mV. The time from symptom onset to intensive care unit admission had to be <48 h. Patients were managed according to usual practice; treatment was not affected by participation in the registry. At the time of admission, when the first routine blood sample was drawn, an additional 50 mL were taken to create DNA and serum banks. The duration of recruitment was 1 month (31 days) per centre for all patients and 2 months for diabetic patients, ranging from 1 October to 24 December, 2005. Of the 374 centres in France that treated patients with acute MI at that time, 223 (60%) participated in the registry. Among these, 100 centres recruited 1029 patients who contributed to both serum and DNA banks. Written informed consent was provided by each patient. Follow-up was collected through contacts with the patients' physicians, the patients themselves or their family, and registry offices of their birthplace. One-year follow-up was >99% complete.
The study was reviewed by the Committee for the Protection of Human Subjects in Biomedical Research of Saint Antoine University Hospital and the data file was declared to the Commission Nationale Informatique et Liberté.

*Blood sampling and measurements*

Blood samples were stored at -80°C at the Department of Clinical Pharmacology, University of Pierre et Marie Curie. All samples were identified by number only and were analysed in random order. Serum concentrations of sPLA$_2$ type IIA were measured by a quantitative and specific time-resolved fluoroimmunoassay (TR-FIA) as previously described.[12] sPLA$_2$ activity was measured using a selective fluorimetric assay as previously described.[6] All samples were tested in duplicate. Serum levels of sPLA$_2$ activity are expressed as nmol/min/mL and sPLA$_2$ IIA mass as ng/mL.

*Genotyping*

Genomic DNA was extracted from whole blood with the MagNA Pure Compact Instrument according to the manufacturer's recommendations (www.roche- applied- science.com) . Five non- synonymous polymorphisms (rs11573156, rs3753827, rs2236771, rs876018, rs3767221) were selected for *PLA2GA* genotyping. All single nucleotide polymorphisms (SNPs) were genotyped with the use of an oligoligation assay (SNPlex, Applied Biosystems, Foster City, CA) after initial polymerase- chain reaction amplification as described previously.[13]

*Statistical analysis*

Outcome events, a composite of all-cause death and non-fatal myocardial infarction (MI), were adjudicated by a committee whose members were unaware of patients' medications, blood measurements, and genotypes.
Baseline demographic and clinical characteristics, treatment factors, and therapeutic management during hospitalisation were compared among the tertile ranges of SPLAY$_2$ activity, with the use of a univariate Cox proportional hazards model. We used a multivariable Cox proportional-hazards model to assess the independent prognostic value of variables with the outcome events during the 1-year follow-up period. The multivariable model comprised sex, age, previous or current smoking, family history of coronary disease, history of hypertension, acute MI, heart failure, renal failure, diabetes, blood pressure at admission, heart rate at admission, Killip class, left ventricular ejection fraction, prior angioplasty or coronary artery bypass surgery, hospital management (including reperfusion therapy, statins, beta-blockers, aspirin, clopidogrel, diuretics, digitalis glycosides, heparin), tertiles of $sPLA_2$ activity, and $sPLA_2$ IIA mass. Serum levels of $sPLA_2$ activity and $sPLA_2$ IIA mass were log-transformed to remove positive skewness. Results are expressed as hazard ratios for Cox models with 95% confidence intervals (CIs). All statistical tests were two-sided and performed using SAS software version 9.1.

### Haplotypic analysis

The Hardy-Weinberg equilibrium and the linkage disequilibrium (D') of the different SNPs were assessed using the Haploview program.[14] Haplotype analyses were performed using the THESIAS software as described previously.[15] Haplotype frequencies were estimated assuming Hardy-Weinberg equilibrium at the haplotypic level. Haplotypes with frequencies <0.05 were not used for further analysis. Haplotypic association effects, expressed as mean quantitative effects for $sPLA_2$ activity and $sPLA_2$ IIA levels or as odds ratios (ORs) for clinical outcomes, were estimated by comparison with a reference haplotype chosen as the most frequent one.

The relationship between the phenotype and haplotypes was modelled using a regression linear model for $sPLA_2$ activity and sPLA IIA levels and a regression model for cardiovascular outcome. Models assumed additive effects of haplotypes on phenotype. A global test of association between haplotypes and the phenotype was performed by a likelihood ratio test. Because of multiple testing, the significance level was taken as P<0.01.[16,17]

### Role of the funding source

The sponsors had no involvement in the collection, analysis, and interpretation of data; in the writing of the report; or in the decision to submit the paper for publication.

### Results

### Baseline demographics and clinical presentation

Patients who had an outcome event during 1 year follow-up were older (75 ± 12 vs 65 ± 13 years) than those without an outcome event and a smaller proportion were male (60% vs 72%) (table 1). They also had a higher rates of hypertension (80% vs 60%), diabetes (50% vs 29%), prior myocardial infarction (25% vs 16%), and heart or renal failure (both 15% vs 4%). In addition, patients who had an outcome were less likely to be treated during the first 48 hours of admission with statins (67% vs 81%), beta-blockers (49% vs 75%), clopidogrel (79% vs 90%), or unfractionated heparin (76% vs 84%) but more often received diuretics (63% vs 30%) and digitalis treatment (5% vs 2%) (table 1).

*Table 1:* **Patient baseline characteristics and in-hospital management**

| | Patients without outcome event (n=893) | Patients with outcome event (n=136) | p* |
|---|---|---|---|
| Demographic | | | |
| Men | 643 (72%) | 81 (60%) | 0·003 |
| Age (years) | 65 ± 13 | 75 ± 12 | <0·001 |
| Medical history | | | |
| Hypertension | 533 (60%) | 108 (80%) | <0·001 |
| Hypercholesterolaemia | 469 (53%) | 71 (52%) | 0·94 |
| Diabetes mellitus | 259 (29%) | 68 (50%) | <0·001 |
| Family history of CAD | 234 (26%) | 18 (13%) | 0·001 |
| Previous or current smoker | 510 (57%) | 57 (42%) | 0·001 |
| Heart failure | 32 (4%) | 20 (15%) | <0·0001 |
| Myocardial infarction | 145 (16%) | 34 (25%) | 0·015 |
| PCI or CABG | 150 (17%) | 28 (21%) | 0·34 |

(continued)

| Medical history | | | |
|---|---|---|---|
| Stroke or transient ischaemic attack | 61 (7%) | 19 (14%) | 0·004 |
| Chronic renal failure | 32 (4%) | 20 (15%) | <0·0001 |
| Clinical presentation | | | |
| Body mass index (kg/m$^2$) | 27 $\pm$ 5 | 26 $\pm$ 5 | 0·090 |
| Systolic BP at admission (mmHg) | 141 $\pm$ 28 | 140 $\pm$ 31 | 0·56 |
| Diastolic BP at admission (mmHg) | 81 $\pm$ 17 | 77 $\pm$ 17 | 0·015 |
| Heart rate at admission (bpm) | 78 $\pm$ 20 | 86 $\pm$ 23 | <0·0001 |
| ST-segment elevation | 478 (54%) | 53 (39%) | 0·002 |
| myocardial infarction | | | |
| Killip class II-IV | 174 (20%) | 65 (48%) | <0·0001 |
| GRACE risk score | 159 $\pm$ 36 | 186 $\pm$ 35 | <0·0001 |
| Left ventricular ejection fraction | 53 $\pm$ 12 | 46 $\pm$ 14 | <0·0001 |
| Baseline biological exam | | | |
| sPLA$_2$ activity (nmol/mL/min) | 3.4 $\pm$ 3 | 4.8 $\pm$ 4 | <0·0001 |
| SPLA$_2$ type IIA mass (ng/mL) | 44 $\pm$ 217 | 53 $\pm$ 100 | 0·001 |
| C-reactive protein (mg/L) | 10.7 $\pm$ 14.5 | 15.8 $\pm$ 18.5 | <0·0001 |
| In-hospital management | | | |
| PCI | 636 (71%) | 59 (43%) | <0·0001 |
| Thrombolysis | 152 (17%) | 11 (8%) | 0·002 |
| Coronary artery bypass surgery | 35 (4%) | 4 (3%) | 0·53 |
| Statin | 721 (81%) | 91 (67%) | 0·0001 |
| Beta-blocker | 668 (75%) | 67 (49%) | <0·0001 |
| Calcium channel blocker | 180 (20%) | 36 (26%) | 0·13 |
| ACE inhibitor or ARB | 488 (55%) | 70 (51%) | 0·45 |
| Nitrate derivative | 453 (51%) | 81 (60%) | 0·052 |
| Aspirin | 822 (92%) | 119 (88%) | 0·051 |
| Clopidogrel | 805 (90%) | 107 (79%) | <0·001 |
| Heparin | 748 (84%) | 103 (76%) | 0·014 |
| Low-molecular-weight heparin | 580 (65%) | 69 (51%) | 0·001 |

(continued)

| In-hospital management | | | |
|---|---|---|---|
| Diuretic | 261 (30%) | 85 (63%) | <0·0001 |
| Glycoprotein IIb/IIIa inhibitor | 368 (41%) | 44 (32%) | 0·063 |
| Digitalis glycoside | 13 (2%) | 7 (5%) | 0·003 |
| Data are n (%) or mean (SD). ACE: angiotensin-converting enzyme inhibitors; ARB: angiotensin receptor blocker; BP: blood pressure; PCI: percutaneous coronary intervention; CABG: coronary artery bypass graft. *Cox univariate analysis | | | |

The baseline characteristics of patients also varied according to $sPLA_2$ activity, as shown in table 2. Patients in the highest tertile were older, with a higher frequency of previous hypertension, diabetes, myocardial infarction, stroke or transient ischaemic attack, heart failure, and chronic renal failure. In-hospital, they less frequently received reperfusion therapy (percutaneous coronary intervention or thrombolysis), statins, beta-blockers, clopidogrel, and heparin treatments, but more frequently received diuretics and digitalis.

*Table 2:* **Baseline characteristics and hospital management of patients according to $sPLA_2$ tertile**

| $sPLA_2$ activity (nmol/mL/min) | [0 - 2.095[ | [2.095 - 3.15[ | ≥3.15 | p* |
|---|---|---|---|---|
| | (n=321) | (n=335) | (n=373) | |
| Demographic | | | | |
| Men | 256 (80%) | 235 (70%) | 233 (63%) | 0.003 |
| Age (years) | 65 ± 13 | 65 ± 14 | 69 ± 14 | <0·001 |
| Medical history | | | | |
| Hypertension | 199 (62%) | 184 (55%) | 258 (69%) | <0·001 |
| Hypercholesterolaemia | 166 (52%) | 172 (51%) | 202 (54%) | 0·94 |
| Diabetes mellitus | 75 (23%) | 96 (29%) | 156 (42%) | <0·001 |
| Family history of CAD | 74 (23%) | 100 (30%) | 78 (21%) | 0·001 |
| Previous or current smoker | 190 (59%) | 198 (59%) | 179 (48%) | 0·001 |
| Myocardial infarction | 47 (15%) | 62 (19%) | 70 (19%) | 0·015 |
| PCI or CABG | 55 (17%) | 61 (18%) | 62 (17%) | 0·34 |
| Stroke or transient ischaemic attack | 24 (7%) | 21 (6%) | 35 (9%) | 0·004 |
| Heart failure | 12 (4%) | 13 (4%) | 27 (7%) | <0·0001 |
| Chronic renal failure | 10 (3%) | 13 (4%) | 29 (8%) | <0·0001 |
| Clinical presentation | | | | |
| Body mass index (kg/m$^2$) | 27 ± 4 | 27 ± 5 | 27 ± 5 | 0·09 |
| Systolic BP at admission (mmHg) | 141 ± 29 | 140 ± 27 | 140 ± 29 | 0·56 |
| Diastolic BP at admission (mmHg) | 81 ± 18 | 80 ± 16 | 80 ± 17 | 0·015 |
| Heart rate at admission (bpm) | 77 ± 23 | 78 ± 18 | 83 ± 21 | <0·0001 |
| ST-segment elevation myocardial infarction | 168 (52%) | 168 (50%) | 195 (52%) | 0.002 |

(continued)

| Clinical presentation | | | | |
|---|---|---|---|---|
| Killip class II-IV | 52 (16%) | 70 (21%) | 117 (32%) | <0·0001 |
| GRACE risk score | 158 ± 35 | 158 ± 36 | 172 ± 38 | <0·0001 |
| Left ventricular ejection fraction | 54 ± 11 | 54 ± 13 | 49 ± 12 | <0·0001 |
| Baseline biological exam | | | | |
| C-reactive protein (mg/L) | 6.8 ± 12 | 8.6 ± 14 | 17.7 ± 17 | 0·0003 |
| sPLA$_2$ type IIA mass (ng/mL) | 15 ±124 | 11.5 ± 21 | 102 ± 314 | 0·0012 |
| In-hospital management | | | | |
| PCI | 242 (75%) | 225 (67%) | 228 (61%) | <0·0001 |
| Thrombolysis | 56 (18%) | 58 (17%) | 49 (13%) | 0·002 |
| CABG | 11 (3%) | 14 (4%) | 14 (4%) | 0·53 |
| Statin | 256 (80%) | 268 (80%) | 288 (77%) | 0·0001 |
| Beta-blocker | 236 (74%) | 250 (75%) | 249 (67%) | <0·0001 |
| Calcium channel blocker | 81 (25%) | 70 (21%) | 65 (17%) | 0·13 |
| ACE inhibitor or ARB | 180 (56%) | 159 (48%) | 219 (59%) | 0·45 |
| Nitrate derivative | 155 (48%) | 170 (51%) | 209 (56%) | 0·052 |
| Aspirin | 299 (94%) | 305 (91%) | 337 (90%) | 0·051 |
| Clopidogrel | 294 (92%) | 294 (88%) | 324 (87%) | <0·0001 |
| Heparin | 268 (83%) | 279 (83%) | 304 (82%) | 0·014 |
| Low-molecular-weight heparin | 217 (68%) | 213 (64%) | 219 (59%) | 0.001 |
| Diuretic | 84 (26%) | 91 (27%) | 171 (46%) | <0.0001 |
| Glycoprotein IIb/IIIa inhibitor | 146 (45%) | 125 (37%) | 141 (38%) | 0·063 |
| Digitalis glycosides | 3 (1%) | 6 (2%) | 11 (3%) | 0·003 |
| Data are n (%) or mean (SD). ACE: angiotensin-converting enzyme inhibitors; ARB: angiotensin receptor blocker; BP: blood pressure; PCI: percutaneous coronary intervention; CABG: coronary artery bypass graft. *Cox univariate analysis | | | | |

## sPLA$_2$ activity and clinical outcomes at 1 year

Of the 1029 patients enrolled, 136 (13·2%) patients died or had a myocardial infarction during the 1- year follow-up period. The probability of outcome events as a function of baseline sPLA$_2$ activity is presented in figure 1. At 1 year, the event rate for death and MI increased across tertiles of sPLA$_2$ activity (tertile 1: n=25, 8%; tertile 2: n=38, 11%; tertile 3: n=73, 20%) . The respective hazard ratios for event rates in the second and third tertiles of sPLA$_2$ activity were 1·54 (95% confidence interval [CI] 0·93- 2·55) and 2·72 (95% CI 1·73- 4·29), compared with the lowest tertile (p<0·0001) .
After adjustment for known cardiovascular risk factors, C-reactive protein, sPLA$_2$ type IIA levels, and treatments including statins, sPLA$_2$ activity remained an independent correlate of the risk of death or MI. The hazards of death or recurrent acute MI with increasing tertile of sPLA$_2$ activity were 1·62 (95% CI 0·96-2·72) and 1·92 (95% CI 1·17-3·17) compared with the first tertile (p=0·037).

## *PLA2G2A* haplotypes and sPLA$_2$ activity

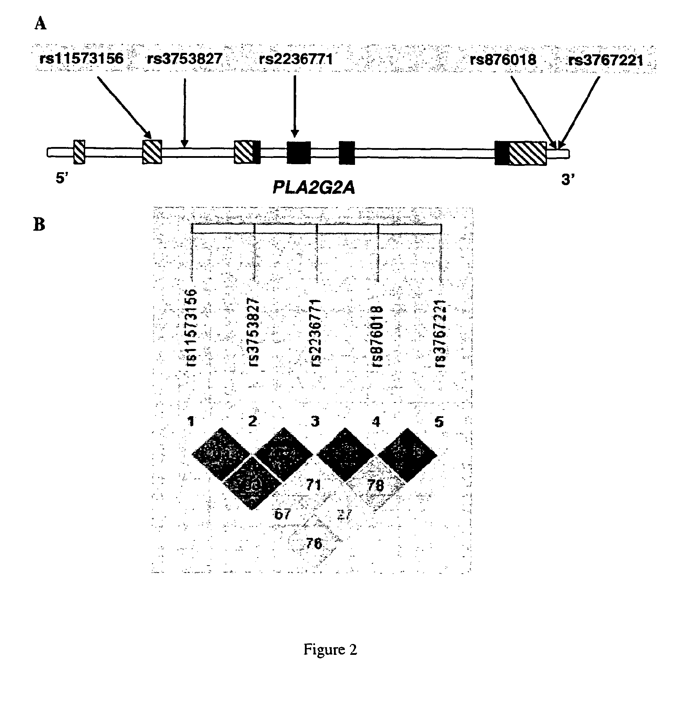

The genotype distribution of the five SNPs for the *PLA2G2A* gene (rs11573156, rs3753827, rs2236771, rs876018, rs3767221) was consistent with the Hardy-Weinberg equilibrium (observed allele frequencies are listed in table 3). Their location is presented in figure 2, together with their pair-wise linkage disequilibrium.

**Table 3: Distribution of minor allele frequencies of the single nucleotide polymorphisms for the *PLA2G2A* gene**

| rs number | Location (NCBI Build 36.3) | Nucleotide change* | Minor allele frequency | p(HWE) exact |
|---|---|---|---|---|
| rs11573156 | 20 178 733 | C/<u>G</u> | 0·207 | 0·09 |
| rs3753827 | 20 178 474 | <u>A</u>/C | 0·464 | 0·30 |
| rs2236771 | 20 177 549 | <u>C</u>/G | 0·084 | 0·49 |
| rs876018 | 20 174 514 | A/<u>T</u> | 0·151 | 0·16 |
| rs3767221 | 20 174 368 | A/<u>C</u> | 0·416 | 0·32 |
| (*minor allele outlined) | | | | |

By single locus analysis, rs11573156 C/G, rs3753827 A/C, rs2236771 C/G, and rs3767221 A/C polymorphisms were significantly associated with concentration levels of $sPLA_2$ IIA and $sPLA_2$ activity. Among them, the rs11573156 G allele was associated with elevated $sPLA_2$ activity, whereas the rs3753827 A, rs2236771 C, and rs3767221 C alleles were linked with a decrease in $sPLA_2$ activity (p <$10^{-6}$, p=0·005, p=0·00008, p<$10^{-6}$, respectively).

Of the 30 potential haplotypes defined by the five SNPs, 16 inferred haplotypes were observed in the sample. Seven of these occurred at frequencies ≥5% and accounted for 94% of the observed haplotypes. Table 4 shows the association of these haplotypes with $sPLA_2$ activity, giving the mean value for one copy of each haplotype as determined by THESIAS.[15] Overall, haplotypic variation in *PLA2G2A* was associated with a significant effect on sPLA2 activity (p<0·0001) . After applying correction for multiple testing, the GCGAA haplotype was the single haplotype that remained significantly associated with variations in sPLA2 activity. This haplotype, the only one carrying the rs11573156 G allele, was associated with 34- 5% increase in sPLA2 activity compared with the most frequent CAGAA haplotype chosen as the reference (2·22 [2·1- 2·36] vs 1·65 nmol/mL/min [1·55- 1·75], p<0·0001) . The CCCAC haplotype, the only haplotype carrying the rs2236771 C allele, was associated with a trend towards a decrease (15·8%) in sPLA2 activity compared with the CAGAA haplotype (1·39 [1·24- 1·54] vs 1- 65 nmol/mL/min [1·55- 1·75], p=0·01) .

***Table 4:* Frequency and associated $sPLA_2$ activity for *PLA2G2A* single nucleotide polymorphism haplotypes***

| Haplotype | Frequency | $sPLA_2$ activity† (nmol/mL/min) | p value vs CAGAA‡ |
|---|---|---|---|
| CAGAA | 0·193 | 1·65 ± 0.10 | |
| CCGAC | 0·186 | 1·56 ± 0·10 | 0·22 |
| <u>GCGAA</u> | 0·17 | 2.22 ± 0·12 | <$10^{-6}$ |
| CAGAC | 0·138 | 1·55 ± 0·13 | 0·25 |
| CAGTA | 0·126 | 1·74 ± 0·12 | 0·25 |
| CCCAC | 0·072 | 1·39 ± 0·15 | 0·01 |
| CCGAA | 0·055 | 1·86 ± 018 | 0·06 |
| *Occurring at frequencies ≥5% and accounting for 94% of the observed haplotypes. †Mean value for one copy of haplotype. ‡Overall p<0.0001. | | | |

### *PLA2G2A* haplotypes and clinical outcomes at 1 year

The GCGAA haplotype was the only haplotype associated with the risk of death and recurrent MI during the first year of follow-up. After adjustment for age, sex, diabetes, hypertension, C-reactive protein, and early use of statins, the OR for outcome events as a function of the GCGAA haplotype remained significant (1·84, 95% CI 1·14-2·95, p=01). As expected from a causal association, additional adjustment for serum levels of $sPLA_2$ type IIA mass and $sPLA_2$ activity abolished the association between GCGAA haplotype and risk of death and recurrent MI (OR 1·33, 95% CI 0·81-2·18, p=0·25).

The OR of death and recurrent MI was reduced for the CCCAC haplotype, the only haplotype associated with a trend towards a decrease in $sPLA_2$ activity, but the reduction was not significant compared with the reference CAGAA haplotype (OR 0·61, 95% CI 0·26-1·48, p=0·27).

**References**

1. Lambeau G, Gelb MH. Biochemistry and physiology of mammalian secreted phospholipases A2. Annu Rev Biochem 2008; 77: 495-520.

2. Hurt-Camejo E, Camejo G, Peilot H, Oomi K, Kovanen P. Phospholipase A(2) in vascular disease. Circ Res 2001; 89: 298-304.

3. Webb NR. Secretory phospholipase A2 enzymes in atherogenesis. Curr Opin Lipidol 2005; 16: 341-44.

4. Jonsson-Rylander AC, Lundin S, Rosengren B, Pettersson C, Hurt-Camejo E. Role of secretory phospholipases in atherogenesis. Curr Atheroscler Rep 2008; 10: 252-59.

5. Boekholdt SM, Keller TT, Wareham NJ, et al. Serum Levels of Type II Secretory Phospholipase A2 and the Risk of Future Coronary Artery Disease in Apparently Healthy Men and Women. The EPIC-Norfolk Prospective Population Study. Arterioscler Thromb Vasc Biol 2005; 25: 839-46.

6. Mallat Z, Benessiano J, Simon T, et al. Circulating secretory phospholipase A2 activity and risk of incident coronary events in healthy men and women. The EPIC-NORFOLK Study. Arterioscler Thromb Vasc Biol 2007; Jan 25 [Epub ahead of print]**.**

7. Kugiyama K, Ota Y, Takazoe K, et al. Circulating levels of secretory type II phospholipase A(2) predict coronary events in patients with coronary artery disease. Circulation 1999; 100: 1280-84.

8. Mallat Z, Steg PG, Benessiano J, et al. Circulating secretory phospholipase A2 activity predicts recurrent events in patients with severe acute coronary syndromes. J Am Coll Cardiol 2005; 46: 1249-57.

11. Cambou JP, Simon T, Mulak G, Bataille V, Danchin N. The French registry of Acute ST elevation or non-ST-elevation Myocardial Infarction (FAST-MI): study design and baseline characteristics. Arch Mal Coeur Vaiss 2007; 100: 524-34.

12. Nevalainen TJ, Eerola LI, Rintala E, Laine VJ, Lambeau G, Gelb MH. Time-resolved fluoroimmunoassays of the complete set of secreted phospholipases A2 in human serum. Biochim Biophys Acta 2005; 1733: 210-23.

13. Philippi A, Roschmann E, Tores F, et al. Haplotypes in the gene encoding protein kinase c-beta (PRKCB1) on chromosome 16 are associated with autism. Mol Psychiatry 2005; 10: 950-60.

14. Barrett JC, Fry B, Maller J, Daly MJ. Haploview: analysis and visualization of LD and haplotype maps. Bioinformatics 2005; 21: 263-65.

15. Tregouet DA, Escolano S, Tiret L, Mallet A, Golmard JL. A new algorithm for haplotype-based association analysis: the Stochastic-EM algorithm. Ann Hum Genet 2004; 68: 165-77.

16. Rothman KJ. No adjustments are needed for multiple comparisons. Epidemiology 1990; 1: 43-46.

17. Perneger TV. Adjusting for multiple testing in studies is less important than other concerns. BMJ 1999; 318: 1288.

**Claims**

1. A method of identifying a subject having or at risk of having or developing a cardiovascular disease and/or a cardiovascular event, comprising determining, in a sample obtained from said subject, the presence or absence of the sPLA2 type IIA haplotype comprising variant alleles in rs11573156 SNP,
   wherein

   - haplotype wherein the variant allele (G) in SNP rs11573156 is present indicates an increased risk of having or being at risk of having or developing a cardiovascular disease and/or cardiovascular event, said haplotype being GCGAA for SNPs rs11573156, rs3753827, rs2236771, rs876018 and rs3767221 respectively.

2. The method according to claim **1**, wherein the presence or absence of said SNP is determined by nucleic acid sequencing or by PCR analysis.

3. The method according to claim **1** or **2**, wherein said cardiovascular disease and/or cardiovascular event is Metabolic Syndrome, Syndrome X, atherosclerosis, atherothrombosis, coronary artery disease, stable and unstable angina pectoris, stroke, diseases of the aorta and its branches (such as aortic thrombosis or aortic aneurysm), peripheral vascular disease, cerebrovascular disease, and any acute ischemic cardiovascular event.

**Patentansprüche**

1. Verfahren zur Identifizierung eines Individuums, welches eine kardiovaskuläre Krankheit und/oder einen kardiovaskulären Vorfall hat oder gefährdet ist eine/einen zu haben oder zu entwickeln, umfassend Bestimmen des Vorhandenseins oder Nichtvorhandenseins des sPLA2 Typ IIA Haplotyps, umfassend abweichende Allele in rs11573156 SNP, in einer von dem Individuum erhaltenen Probe,
   worin

   - Haplotyp, worin die Allelvariante Allel (G) in SNP rs11573156 vorhanden ist, auf ein erhöhtes Risiko eine kardiovaskuläre Krankheit und/oder einen kardiovaskulären Vorfall zu haben oder gefährdet zu sein eine/einen zu haben oder zu entwickeln hinweist, wobei der Haplotyp für SNPs rs11573156, rs3753827, rs2236771, rs876018 beziehungsweise rs3767221 GCGAA ist.

2. Verfahren nach Anspruch 1, wobei das Vorhandensein oder Nichtvorhandensein des SNPs durch Nukleinsäuresequenzierung oder durch PCR-Analyse bestimmt wird.

3. Verfahren nach Anspruch 1 oder 2, wobei die kardiovaskuläre Krankheit und/oder der kardiovaskuläre Vorfall metabolisches Syndrom, Sydrom X, Arteriosklerose, Atherothrombose, koronare Arterienkrankheit, stabile und instabile Angina pectoris, Schlaganfall, Erkrankung der Aorta und ihrer Verzweigungen (wie zum Beispiel aortische Thrombose oder aortisches Aneurysma), periphere Gefäßerkrankung, cerebrovaskuläre Erkrankung und irgendein akuter ischämischer kardiovaskulärer Vorfall.

**Revendications**

1. Procédé d'identification d'un sujet ayant ou risquant d'avoir ou de développer une maladie cardiovasculaire et/ou un évènement cardiovasculaire, comprenant la détermination, dans un échantillon obtenu dudit sujet, de la présence ou de l'absence de l'haplotype sPLA2 de type IIA comprenant des allèles variants dans rs11573156 SNP,
   dans lequel

   - l'haplotype dans lequel l'allèle variant (G) dans le SNP rs11573156 est présent indique un risque accru d'avoir ou de risquer d'avoir ou de développer une maladie cardiovasculaire et/ou un évènement cardiovasculaire, ledit haplotype étant GCGAA pour les SNP rs11573156, rs3753827, rs2236771, rs876018 et rs3767221, respectivement.

2. Procédé selon la revendication 1, dans lequel la présence ou l'absence dudit SNP est déterminée par séquençage d'acide nucléique ou par une analyse par PCR.

3. Procédé selon la revendication 1 ou 2, dans lequel ladite maladie cardiovasculaire et/ou ledit évènement cardiovasculaire est le syndrome métabolique, le syndrome X, l'athérosclérose, l'athérothrombose, une coronaropathie, l'angine de poitrine stable et instable, un AVC, des maladies de l'aorte et de ses ramifications (telles que la thrombose aortique ou un anévrisme aortique), une maladie vasculaire périphérique, une maladie cérébrovasculaire et tout évènement cardiovasculaire ischémique aigu.

Figure 1

**A**

**B**

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008015546 A

### Non-patent literature cited in the description

- **RADVANYI et al.** *Anal Biochem,* 1989, vol. 177, 103-9
- **PERNAS et al.** *Biochem Biophys Res Commun,* 1991, vol. 178, 1298-1305
- **LAMBEAU G ; GELB MH.** Biochemistry and physiology of mammalian secreted phospholipases A2. *Annu Rev Biochem,* 2008, vol. 77, 495-520
- **HURT-CAMEJO E ; CAMEJO G ; PEILOT H ; OOMI K ; KOVANEN P.** Phospholipase A(2) in vascular disease. *Circ Res,* 2001, vol. 89, 298-304
- **WEBB NR.** Secretory phospholipase A2 enzymes in atherogenesis. *Curr Opin Lipidol,* 2005, vol. 16, 341-44
- **JONSSON-RYLANDER AC ; LUNDIN S ; ROSENGREN B ; PETTERSSON C ; HURT-CAMEJO E.** Role of secretory phospholipases in atherogenesis. *Curr Atheroscler Rep,* 2008, vol. 10, 252-59
- **BOEKHOLDT SM ; KELLER TT ; WAREHAM NJ et al.** Serum Levels of Type II Secretory Phospholipase A2 and the Risk of Future Coronary Artery Disease in Apparently Healthy Men and Women. The EPIC-Norfolk Prospective Population Study. *Arterioscler Thromb Vasc Biol,* 2005, vol. 25, 839-46
- **MALLAT Z ; BENESSIANO J ; SIMON T et al.** Circulating secretory phospholipase A2 activity and risk of incident coronary events in healthy men and women. The EPIC-NORFOLK Study. *Arterioscler Thromb Vasc Biol,* 25 January 2007
- **KUGIYAMA K ; OTA Y ; TAKAZOE K et al.** Circulating levels of secretory type II phospholipase A(2) predict coronary events in patients with coronary artery disease. *Circulation,* 1999, vol. 100, 1280-84
- **MALLAT Z ; STEG PG ; BENESSIANO J et al.** Circulating secretory phospholipase A2 activity predicts recurrent events in patients with severe acute coronary syndromes. *J Am Coll Cardiol,* 2005, vol. 46, 1249-57
- **CAMBOU JP ; SIMON T ; MULAK G ; BATAILLE V ; DANCHIN N.** The French registry of Acute ST elevation or non-ST-elevation Myocardial Infarction (FAST-MI): study design and baseline characteristics. *Arch Mal Coeur Vaiss,* 2007, vol. 100, 524-34
- **NEVALAINEN TJ ; EEROLA LI ; RINTALA E ; LAINE VJ ; LAMBEAU G ; GELB MH.** Time-resolved fluoroimmunoassays of the complete set of secreted phospholipases A2 in human serum. *Biochim Biophys Acta,* 2005, vol. 1733, 210-23
- **PHILIPPI A ; ROSCHMANN E ; TORES F et al.** Haplotypes in the gene encoding protein kinase c-beta (PRKCB1) on chromosome 16 are associated with autism. *Mol Psychiatry,* 2005, vol. 10, 950-60
- **BARRETT JC ; FRY B ; MALLER J ; DALY MJ.** Haploview: analysis and visualization of LD and haplotype maps. *Bioinformatics,* 2005, vol. 21, 263-65
- **TREGOUET DA ; ESCOLANO S ; TIRET L ; MALLET A ; GOLMARD JL.** A new algorithm for haplotype-based association analysis: the Stochastic-EM algorithm. *Ann Hum Genet,* 2004, vol. 68, 165-77
- **ROTHMAN KJ.** No adjustments are needed for multiple comparisons. *Epidemiology,* 1990, vol. 1, 43-46
- **PERNEGER TV.** Adjusting for multiple testing in studies is less important than other concerns. *BMJ,* 1999, vol. 318, 1288